# EUROPEAN PATENT SPECIFICATION

(11) **EP 4 346 705 B1**
(45) Date of publication and mention of the grant of the patent: **09.12.2026**
(21) Application number: 22733212.9
(22) Date of filing: 27.05.2022
(51) Int. Cl.: A61F 2/30, A61B 17/86

(54) **KIT FOR THE INSTALLATION OF PROSTHETIC COMPONENTS AND/OR BIOMEDICAL IMPLANTS**
KIT FÜR DIE INSTALLATION VON PROTHETISCHEN KOMPONENTEN UND/ODER BIOMEDIZINISCHEN IMPLANTATEN
KIT POUR L'INSTALLATION DE COMPOSANTS PROTHÉTIQUES ET/OU D'IMPLANTS BIOMÉDICAUX

(30) Priority: 27.05.2021 IT 202100013841
(43) Date of publication of application: 10.04.2024
(73) Proprietor: MT Ortho S.r.l., 95025 Aci Sant'Antonio (IT)
(72) Inventor: DI BELLA, Simone, 95025 ACI Sant'antonio (CT) (IT); ZOCCALI, Carmine, 89127 Reggio Calabria (RC) (IT)
(74) Representative: Bianchetti & Minoja with Trevisan & Cuonzo IPS SRL
(86) International application number: PCT/IB2022/054988
(87) International publication number: WO 2022/249137

(56) References cited:
- WO-A1-2013/150369
- US-A1- 2005 165 493
- US-A1- 2011 166 668
- US-A1- 2014 012 390
- US-B1- 6 284 002
- US-B2- 9 339 394

## Description

### FIELD OF THE INVENTION

The present invention relates to a kit for installation of prosthetic components and/or biomedical implants comprising a prosthetic component having an intraosseous taproot formed of one piece with the prosthesis, for attachment of said prosthetic component to the bone of a patient.

The intraosseous biomedical device for attachment of the prosthetic component, also known in the art as taproot, is made of a biocompatible metal material such as titanium and/or alloys thereof, suitable for use in biomedical implants, namely for attachment of prosthetic components or prosthetic parts, such as, without limitation, orthopedic prostheses, to the bone.

By way of example and without limitation, the kit for installation of prosthetic components and/or biomedical implants of the present invention may be used in the installation of acetabular prosthetic implants, as well as in implantation of prosthetic knees, elbows, shoulders, pelvis, and so on.

### BACKGROUND ART

A number of known biomedical applications use taproots, that are inserted into the bone to firmly fix a prosthetic component to the bone itself.

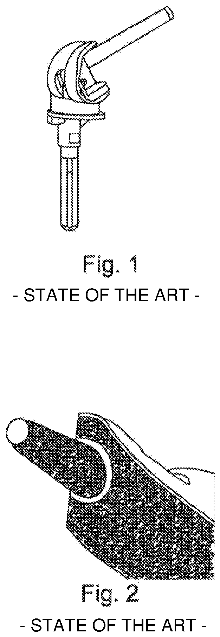

Examples of such applications are found in acetabular prostheses (Fig. 1) as well as knee prostheses (Fig. 2). In all currently known applications, taproots have a circular cross-section, as this shape allows the surgeon to make intraoperative adaptations by rotating the component, for example the cotyl or acetabulum or the tibial tray of a prosthetic knee, about the longitudinal axis of the taproot even when the letter is already inserted in the bone.

Examples of taproots having geometries other than the circular cross-section geometry, include the nails as used for bone fusion, such as for fixation of the sacroiliac joint, or the Thornton Nail, i.e. a three-flange fixation nail that was used in the '70s of the last century for treatment of femoral neck fractures.

An example of a taproot having a triangular geometry is provided in US 9 339 394 B2, which illustrates a prosthetic vertebral facet suitable for replacement of cartilage and a bone portion of the natural vertebral joint facet. Among alternative embodiments of the fixation system, a transpedicular screw having a triangular cross-section is shown. Therefore, the taproot of this document is not rigidly joined with the prosthetic element, whereby the patent does not imply the problem of ensuring proper orientation of the prosthetic element in space in response to the orientation of the taproot, and does not show or suggests the use of guide components for proper positioning of the prosthetic element,.

No example of systems or kits are currently available which use taproots with a section other than the cylindrical section in intraosseous fixation systems, for reconstruction/replacement of bone parts, because the circular section can roughly fit any anatomy, and can thus afford series production in different sizes, with the surgeon having the responsibility for optimized positioning of the taproot in the bone of the patient, and thus for orientation of the prosthetic element associated with the taproot.

Nevertheless, these known arrangements still suffer from certain drawbacks.

These drawbacks mainly include the inability of conventional circular-section taproots to resist torsional loads on the prosthesis. A stem having a cylindrical section opposes low resistance to torsional strain, only provided by the friction between the stem and the inner surfaces of the bone contacted by said stem.

Furthermore, prior art circular section-taproots leave responsibility to the surgeon, who has no reference and shall use fluoroscopy, which in fact exposes the surgeon him/herself and the patient to X-rays
Therefore, the present patent application has the purpose to obviate the residual drawbacks of the prior art, as more clearly discuss4ed hereinafter.

### SUMMARY OF INVENTION

The main purpose of the present invention is to provide a kit for installation of prosthetic components and/or biomedical implants, wherein said prosthetic component comprises a taproot for intraosseous fixation of the prosthetic component in reconstruction and/or replacement of bone parts, which can solve or at least reduce the drawbacks of prior art systems.

In the pursuance of this purpose, an object of the present invention is to provide a prosthetic component comprising a taproot for intraosseous fixation of the prosthetic component, that is able to withstanding torsional loads.

A further object of the present invention is to provide a prosthetic component comprising a taproot for intraosseous fixation of the prosthetic component, that affords unique positioning of the taproot and of the prosthetic component associated therewith relative to the anatomical site of the patient with which the prosthesis is to be associated, thereby guiding the surgeon in positioning the taproot and hence the prosthetic component, and dramatically reducing the risk of wrong positioning and optimizing the surgery, thereby affording bone reconstruction even in highly complex situations.

The above purposes and these and other objects of the present invention are fulfilled by a kit for installation of prosthetic components and/or biomedical systems, wherein said prosthetic component comprises a taproot for intraosseous fixation of the prosthetic component in reconstruction and/or replacement of bone parts, thereby affording superior stability of the implant and excellent load-bearing capacity even after a short time from reconstruction.

The above purpose and these and other objects of the present invention are fulfilled by a kit for installation of prosthetic components comprising a taproot for intraosseous fixation as defined in claim 1.

Further characteristics of the kit and the prosthetic component comprising a taproot for intraosseous fixation according to the present invention will form the subject of the dependent claims.

### LIST OF FIGURES

The characteristics and advantages of the kit for installation of prosthetic components and/or biomedical implants comprising a taproot made of a biocompatible metal material for the intraosseous fixation of said prosthetic component according to the present invention and of the prosthetic component comprising said taproot according to the present invention will be more readily apparent upon reading of the following detailed description, given by way of example and without limitation, with reference to the accompanying schematic drawings, in which:
- Figures 1 and 2 show examples of prior art circular-section taproots;
- Figure 3 shows a front view of the taproot which equips the prosthetic components of the present invention;
- Figure 4 shows a front view of the taproot of Figure 3, which shows the central body only, without the trabeculated portion;
- Figure 5 is a longitudinal sectional view as taken along a vertical plane A-A as shown in Figure 4;
- Figure 6 is a top plan view of the taproot of Figure 3;
- Figures 7 and 8 show perspective views of the taproot of the present invention which equips an acetabular prosthetic component;
- Figure 9A shows a front view of an example of a first guide wire-guiding element according to an embodiment suitable for installation of an acetabular prosthetic component;
- Figure 9B is a perspective view of the guide wire-guiding element of Figure 9B;
- Figure 9C shows a step of Computer Tomography imaging of the surgery zone for defining the design of the guide wire-guiding element;
- Figure 10 shows the guide wire-guiding element of Figures 9A and 9B of the present invention, properly seated and oriented, and associated with an acetabular seat of the pelvis, with the guide wire inserted;
- Figure 11A shows a front view of an example of a second guide element for a triangular impactor;
- Figure 11B is a perspective view of the triangular impactor-guiding element of Figure 9B;
- Figure 11C shows a step of Computer Tomography imaging of the surgery zone for defining the design of the triangular impactor-guiding element;
- Figure 12 shows the triangular impactor-guiding element of Figures 11A and 11B of the present invention, properly seated and oriented, and associated with an acetabular seat of the pelvis;
- Figures 13 and 14 show the taproot of the present invention associated with an inserted acetabular prosthesis associated with the patient's pelvis, with the addition of possible additional fixation screws;
- Figures 15 to 26 show the steps of preparing the surgery site for reconstruction of the left femur-acetabulum joint by means of a fixation kit of the present invention comprising guide components for preparation of the implantation site and a prosthetic element with of a pair of triangular-section taproots according to the present invention.

### DETAILED DESCRIPTION OF THE INVENTION

The present invention relates to a kit **100** for installation of prosthetic cotyloid components **40, 50** in which said prosthetic component **40, 50** comprises one or more taproots **10** depending on the extent of the osteotomy for intraosseous fixation of the prosthetic component itself in reconstruction and/or replacement of bone parts.

The prosthetic component **40, 50** that is part of the kit **100** of the present invention, comprises a taproot **10** for intraosseous fixation, that is formed in one piece with said prosthetic component **40, 50.**

The prosthetic component **40, 50** and the taproot **10** are monolithic constructions made of a biocompatible metal material, more particularly titanium- and/or titanium alloy-based metal materials.

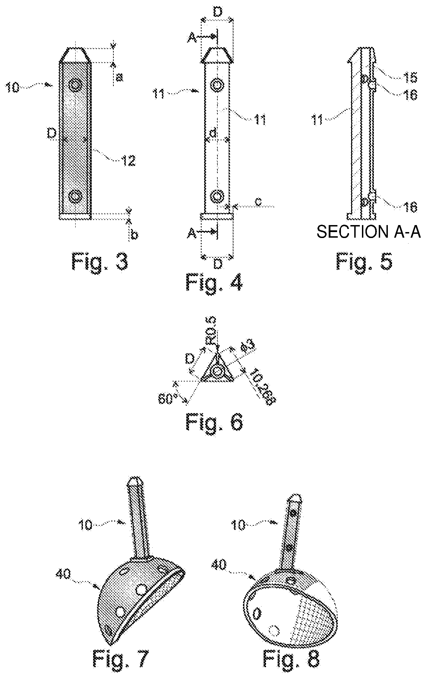

Advantageously, the taproot **10** which equips the prosthetic component **40, 50** of the kit **100** of the present invention, is manufactured using the 3D EBM (Electron Beam melting) additive manufacturing technology and has a structure comprising a solid central body **11** with a triangular cross-section, as viewed in a substantially horizontal plane, and a trabeculated portion **12,** as shown for example in Fig. 3, rigidly joined to said central body **11.**

Advantageously, the taproot **10** of the present invention has at least one portion with a substantially triangular cross section as taken along a horizontal plane. As shown in the top view of Figure 6, and in the perspective views of Figures 7 and 8, according to a preferred embodiment of the present invention, the entire length of the taproot **10** of said prosthetic component **40, 50,** including the end sections **11a, 11b,** has a triangular cross-section.

Advantageously, the taproot **10** of said prosthetic component **40, 50** of the present invention further comprises, inside said central body **11,** a longitudinal through hole **15** for receiving a guide wire during implantation.

Advantageously, the taproot **10** may further comprise one or more additional discharge holes **16** in communication with said axial hole **15,** for putting said axial hole 15 in communication with the outside, in order to create an exit path for any bone cement, platelet concentrate or stem cells injected through the central hole **15** once the latter has been used as a guide for the guide wires, to stabilize the system if the surgeon is not satisfied or confident with the purely mechanical holding strength of the taproot, for example due to the consistency of the bone tissue.

The aforementioned discharge holes **16** may advantageously have an inside diameter of 2.5 mm and an outside diameter of 4.5 mm.

Still referring to the accompanying figures, the taproot **10** of the present invention will comprise, at its front end **11a,** an end element **13** having a frustoconical profile along a vertical plane as shown in Figures 3 to 5, and having a height **a** that is preferably about 5 mm. This frustoconical front end element **13** is configured to facilitate insertion of the taproot into the medullary channel of the patient's bone.

On the other hand, at the rear end **11b** of said taproot **10,** designed for connection to the prosthetic element to be associated with the bone, a base element 14 may be provided, having a width **D** along a vertical longitudinal plane and a height **b** of preferably about 2 mm.

According to a preferred embodiment of the present invention, the taproot **10** is formed in one piece with the prosthetic component **40, 50,** advantageously using the EBM technology.

Advantageously, as shown in the accompanying figures, the aforementioned front end **11a** of said taproot has a frustoconical shape **13** with a proximal larger base **13a** and a distal smaller base **13b.**

Said frustoconical end element **13** is rigidly joined to or, advantageously, formed in one piece by EBM with said central solid core **11,** and the width of its larger base **13a** is equal to the overall width **D** of the taproot.

Advantageously, the width **D** of the larger base **13a** is greater than the width **d** of the cylindrical central core **11,** still considered on a vertical plane like that of the front views of Figures 3 and 4, with the difference **c** = **D-d** indicated in Figure 4 and preferably of more than 1 mm, so that the trabeculated portion **12** formed in one piece by EBM with said central cylindrical body **11** will not exceed the width **D** of said larger base **13a.**

Thus, the trabeculated portion **12** extends around said central body **11** having a triangular cross-section while remaining within the shape defined by the two end elements **13, 14** of the taproot, as shown for example in Figure 3.

By this arrangement, intraosseous insertion is not likely to cause damage to or rupture of the trabeculae.

Figure 6, which shows a top view of the taproot **10** of the present invention, illustrates the triangular cross-sectional configuration of the frustoconical end element **13.**

The axial length of the taproot **10** of the present invention may vary according to the anatomical site and/or the specific needs of the patient, advantageously in a range of 20 mm to 100 mm.

The above described configuration of the taproot **10** of the present invention, which comprises a solid central body **11** and a trabeculated portion **12,** also affords press- or interference-fit insertion of the taproot into the bone during surgery, which provides load resistance, as well as osteointegration over time and stimulation of bone due to the trabeculated portion.

One study (Int. J. Mol. Skiing. 2021, 22, 2379; "Superior Osteo-Inductive and Osteo-Conductive Properties of Trabecular Titanium vs. PEEK Scaffolds on Human Mesenchymal Stem Cells: A Proof of Concept for the Use of Fusion Cages*"*) conducted on titanium scaffolds manufactured by the applicant hereof has confirmed the superior osteoinductive and osteoconductive properties of the Ti6Al4V ELI trabeculated titanium structure.

As mentioned above, the prosthetic component **40, 50** comprising the taproot **10** of the present invention is advantageously formed by manufacturing techniques that include localized melting of (metal or polymer) powders using high-energy electron beams.

These techniques, known as EBM (Electron Beam Melting), are leading-edge manufacturing technologies which can form objects with very complex geometries and different surface roughnesses from a computer design of the finished product, which is processed by computerized machines that guide the electron beam in its action.

Electron beam melting is a relatively new rapid prototyping technique for the production of implant structures, and affords complex three-dimensional geometries.

Using this technique, the Applicant hereof has developed the kit **100** of the present invention, in which the prosthetic component **40, 50** comprises the taproot **10** with the part with the trabeculated structure **12** having pore sizes between trabeculae in the order of one hundred microns.

More particularly, the preferably regular trabeculated structure **12** will have a pore diameter ranging from 400 to 800 microns, more preferably the pore diameter will be about 600 microns, preferably 640 microns.

Namely, the titanium or titanium alloy trabeculated structure **12,** whose elastic modulus is very proximate to that of the natural trabecular bone, restores physiological load transfer, thereby preventing bone damage and even promoting bone regrowth.

It should be noted that the EBM technology allows the trabeculated portion **12** to be formed in one piece with the central body **11.**

The Applicant has also found that the particular configuration of the taproot **10** of the present invention, namely the provision of a central body **11** having a solid structure connected at its ends to the end portions **13, 14** as described, and of the large trabeculated portion **12** extending around said central body **11,** allows the implant to have an optimal mechanical load-bearing behavior *in vivo,* thereby immediately achieving primary stability of the plant, due to both the structure of the taproot **10** and to its triangular cross-section which provides a superior resistance to torsional loads.

Also, the outermost trabeculated portion **12** both ensures osteointegration of the system in the weeks following surgery, and further improves primary stability and "grip", i.e. the friction that opposes movements and in particular taproot withdrawal, since the first postoperative stages.

The taproot 10 of the present invention comprises, as mentioned above, an axial through hole **15,** allowing guided device implantation using a guide wire and, if needed, allowing the surgeon to inject a certain amount of biocement into the taproot to further improve stability of an otherwise simple press-fit.

Also, the triangular cross-section provides significant biomechanical benefits as compared with known taproot types, such as the ability to resist torsional loads, and to facilitate a surgeon, in properly positioning the implant with appropriate instruments relative to the bone, by affording unique positioning, and thus reducing the importance of manual orientation which, in known systems, is defined by the surgeon during surgery, resulting in the above discussed problems.

This specific advantage is achieved by a kit **100** that comprises the taproot **10** of the present invention and additional guide elements forming the instruments that guide the surgeon during prosthesis implantation.

The aforementioned kit **100** is also encompassed by the present invention and comprises, in addition to said taproot **10,** a first guide wire-guiding component **20,** for guiding insertion of a guide wire, and a second guiding component **30** for an impactor having a triangular cross-section.

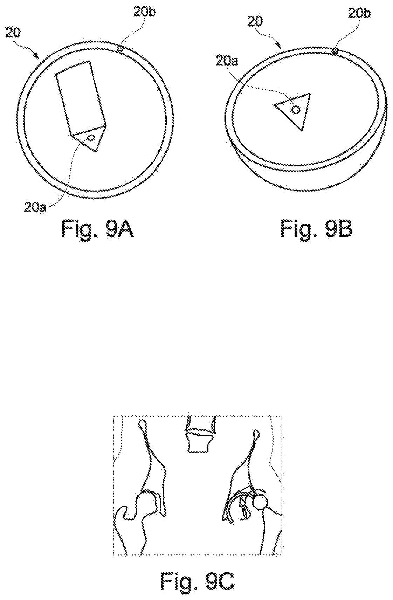

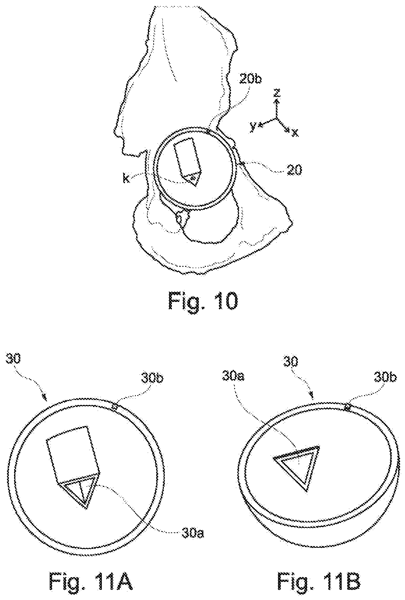

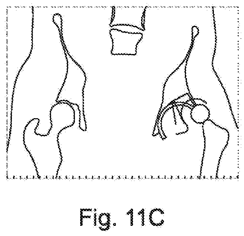

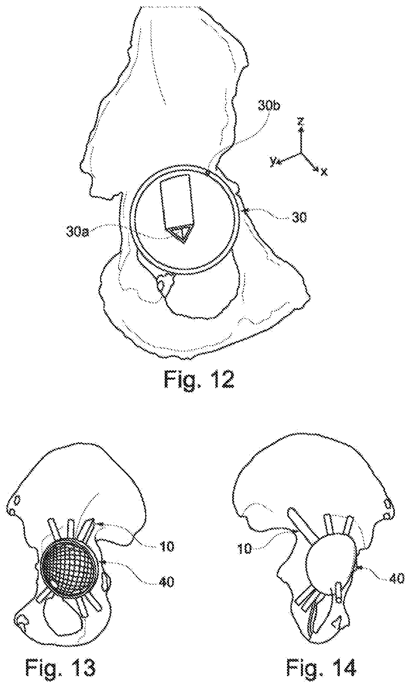

Reference is particularly made to Figures 9A, 9B and 10 which show the first guide wire-guiding component **20,** and Figures 11A, 11B and 12, which show the second guiding component **30** for a triangular impactor adapted to form the seat for the taproot **10** in the bone. As shown in the figures, the first guide wire-guiding component **20** advantageously comprises a seat **20a** for receiving the guide wire **k** and a peripheral marker **20b,** whereas the second triangular impactor-guiding component **30** comprises a first seat **30a** for receiving the guide wires that have been already positioned in the bone and the impactor, and also a peripheral marker **30b.** These references that guide the surgeon in component orientation ensure proper positioning of the guide elements and hence proper orientation of the taproot seat that is formed in the bone for receiving the triangular taproot **10.**

Each of said first and second guiding components **20, 30** is advantageously designed according to the surgical procedure to be performed.

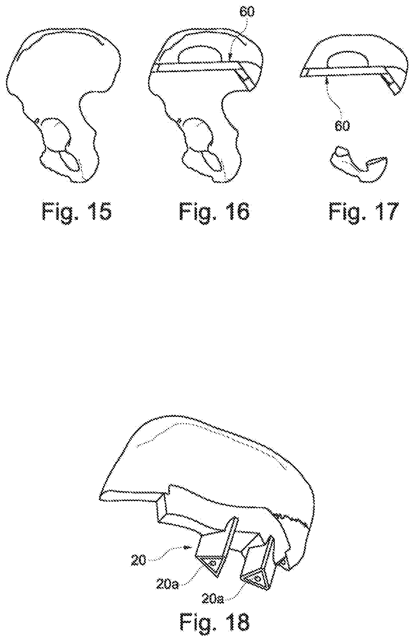

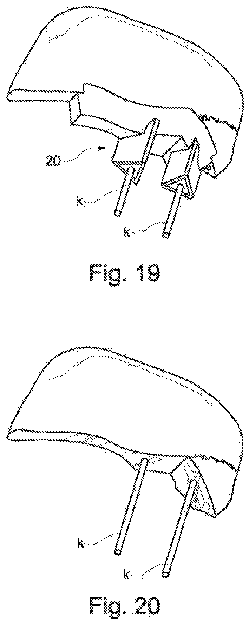

Thus, for example, Figures 18 - 20 show a different embodiment of said first guide wire-guiding component **20,** which is specifically configured for a procedure including, as shown in Figures 15 - 17, osteotomy of the pelvis bone using an anatomical template **60,** such osteotomy possibly covering a large portion of the bone, as may be the unfortunate case of bone cancer.

Said first guide wire-guiding component **20** is configured to perfectly fit the shape of the edge of the bone on which it is designed to rest.

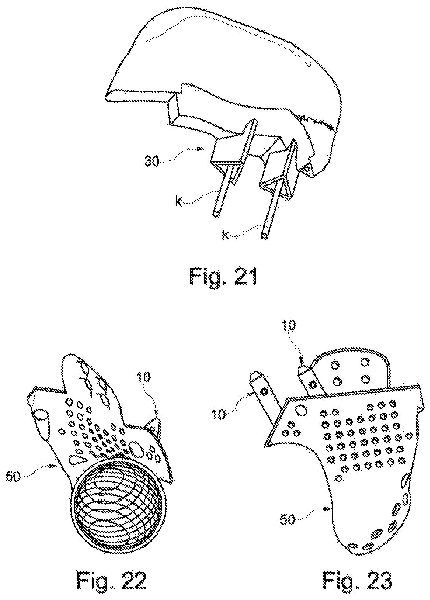

Figure 21 shows a different embodiment of said second guiding component **30** for an impactor having a triangular cross-section, specially designed for the procedure, so as to perfectly fit the shape of the edge of the bone on which it is designed to rest.

In order to manufacture custom-made components that can perfectly fit the bone surfaces to be contacted, to thereby ensure unique, and hence failsafe positioning, said guiding components **20, 30** are also advantageously manufactured with techniques providing localized melting of (metal or polymer) powders using high-energy electron beams, known as EBM.

Thus, said first and second guiding components **20, 30** may be advantageously custom-made to tailor the patient and the type of procedure to be performed, based on a CT scan for imaging the anatomical site in which the prosthesis is to be implanted and planning of the procedure, the guide elements being also accordingly custom-made, for guiding proper positioning of the prosthesis.

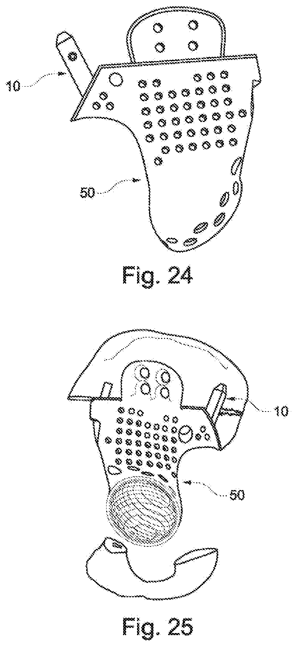

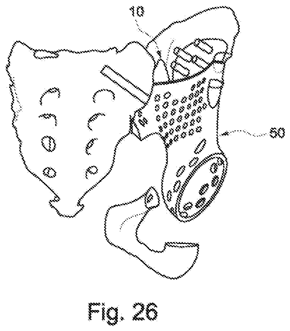

Figures 22 to 26 illustrate by way of example a prosthesis **50** associated with a taproot **10** of the present invention, specifically designed for reconstruction of the femur-acetabular joint as needed to restore the functionality of the joint which had to be completely eliminated due to the selected type of procedure.

While an embodiment of custom-made manufacture of the guiding components **20, 30** will be described below, affording design and performance of even highly complex bone and/or joint reconstruction procedures, such as the one as shown in the accompanying figures, it shall be understood that said taproot **10** and said guiding components **20, 30** may also be mass-produced, advantageously in different sizes, thereby affording the advantage of ensuring superior attachment of the prosthesis to the bone by means of the taproot.

A kit comprising a taproot **10** and custom-made guiding components **20, 30** particularly provides additional benefits as compared with mass-produced components, such benefits being highly appreciated and/or affording procedures that would otherwise be unfeasible with standard mass-produced components.

These benefits, as discussed above, may be summarized as follows:
- very high shape precision to ensure the highest precision in the contact surfaces between the prosthesis and the bone;
- no need to change or adapt positioning of the components (taproot and guide components) during the procedure, which allows the surgeon to be assured of proper prosthesis positioning even under very difficult procedure conditions;
- excellent biointegrative and osteoinductive properties, due to the trabeculated structure of the taproot and high mechanical resistance also to torsional loads due to the solid central portion and the triangular section;
- radiopacity and low infection risk rate.

In order to produce custom-made components, the captured images are processed by three-dimensional image processing software and the design of the custom-made guiding components **20, 30** is made.

Therefore, the process for custom-made production of the guide wire-guiding component **20** and the impactor-guiding component **30** may advantageously comprise the steps of:
- capturing CT scan images of the patient;
- processing the CT images, followed by segmentation and production of a 3D model of the bone tissue of the patient;
- designing custom-made guiding components **20, 30.**

The above described method of manufacturing the guiding components **20, 30** is focused on restoration of the anatomy and functionality of the anatomical site to be treated, and also affords proper attachment of the taproot 10 and the prosthetic element associated therewith to the bone tissue, thereby ensuring prosthesis stability over time.

The design of specific instruments, namely the guiding components **20, 30,** can guide the surgeon in replicating as faithfully as possible the in-situ positioning of the biomedical device as defined in the pre-operative planning stage, in the operating room.

Such guiding components **20, 30,** as well as the device including the taproot **10** may be designed in a patient-specific manner, as mentioned above, to have mating surfaces perfectly adhering to the host anatomical site, thereby affording the surgeon fixed, unique positioning features, preventing any risk of positioning errors and facilitating the procedure, as the surgeon is not required to change or adapt component positioning during surgery.

The method of using the guiding components **20, 30** according to the invention comprises at least the steps of:
- using said first guiding component **20** to allow insertion of a guide wire **K** in the direction established when designing the surgical procedure;
- then, while holding the guide wire **K** in place, positioning the second guide component **30,** which acts as a guide for proper orientation of an impactor having a triangular section, thereby forming the seat for the taproot **10.**

Successive use of the two guiding components **20, 30** of the invention affords unique definition of the direction and orientation of the taproot **10** and accordingly of the prosthetic component **50,** which is rigidly joined thereto.

Finally, the taproot **10** is implanted in a guided manner, due to the presence of the axial through hole **15** which allows insertion of the guide wire **K,** and is then inserted by interference press-fit into the seat formed in the bone using an impactor guided by said second guiding component **30.**

The kit **100** comprising the taproot **10** configured, as described above, by the two guiding components **20, 30** can replicate during surgery what has been defined in the pre-operative design stage, thus guiding the surgeon in the implantation of the taproot and of the prosthetic component associated therewith.

In case of custom-made prostheses and components, all the components of the kit are designed based on the anatomy of the patient, and in particular based on the morphology of the specific implantation area.

The positioning of the two custom-made guiding components **20, 30** and then of the taproot **10** will be unique as the contact surfaces of the guiding components relative to the bone surface will be unique and perfectly complementary.

The characteristics and advantages of the taproot **10** and the kit **100** comprising such taproot of the present invention will be apparent from the above description.

It was particularly shown that the kit **100** of the present invention, and particularly the guiding elements **20, 30** mass-produced in different sizes or custom-made according to the specific anatomical conformation of the patient, can guide the surgeon in the installation of the triangular taproot **10,** and hence of the associated prosthetic component, with a dramatic reduction or elimination of positioning error risks.

Thus, the dedicated instruments, comprising said guiding elements **20, 30** allow the use of a triangular taproot **10** as described above which, as already mentioned, will provide considerable benefits, including higher resistance to torsional loads and greater precision in positioning the prosthesis to perfectly match with what was established during pre-operative planning.

It shall be understood that the intraosseous triangular taproot thus conceived is susceptible of modifications and/or variants, all of which are encompassed by the invention, whose scope is defined by the accompanying claims.

In particular, the materials described, as well as the dimensions, may vary as needed.

## Claims

1. Kit (100) for the installation of prosthetic components (40, 50) and/or biomedical implants, said kit (100) comprising a prosthetic component (40, 50) in turn comprising, made as one piece, an intraosseous taproot (10) for the attachment of said prosthetic component (40, 50) to the bone of a patient, said prosthetic component (40, 50) and said taproot (10) being made of a biocompatible metal material, particularly titanium or alloys thereof, and wherein said taproot (10) of said prosthetic component (40, 50) comprises a solid central body (11) comprising an axial through-hole (15) and, externally to said central body (11), a trabeculated portion (12) solidly connected to, or made of one piece with, said central body (11), and wherein said taproot (10) presents, in a substantially horizontal plane of cross-section, at least one section having a triangular cross-section, **characterized in that** said kit (100) further comprises at least a first guide component (20) for guide wire to guide the insertion of a guide wire (K) into the bone, and at least a second guide component (30) for an impactor having a triangular cross-section.

2. The kit (100) for the installation of prostheses and/or biomedical implants as claimed in the preceding claim, **characterized in that** each of said guide components (20, 30) is made in a patient-tailored manner, by processing the images from the Computed Tomography scan of the anatomy of the patient.

3. The kit (100) for the installation of prostheses and/or biomedical implants as claimed in the preceding claim, **characterized in that** each of said guide components (20, 30) is designed to uniquely mate the anatomical site of prosthesis implantation, thereby ensuring proper positioning of the guide elements, and hence of the guide wire and the triangular impactor, thereby ensuring proper orientation of the triangular seat inside the bone which in turn ensures proper positioning of the triangular taproot (10).

4. The kit (100) for the installation of prostheses and/or biomedical implants as claimed in the preceding claim, **characterized in that** said prosthetic component (40, 50) and said taproot (10) are made in one piece.

5. The kit (100) for the installation of prostheses and/or biomedical implants as claimed in the preceding claim, **characterized in that** said prosthetic component (40, 50) and said taproot (10) are made in one piece by means of 3D additive techniques,namely EBM technologies.

6. The kit (100) for the installation of prostheses and/or biomedical implants as claimed in one or more of the preceding claims, **characterized in that** said prosthetic element (40, 50) comprises an intraosseous taproot (10) which has a triangular cross-section along its entire length, as viewed in a substantially horizontal plane.

7. The kit (100) for the installation of prostheses and/or biomedical implants as claimed in one or more of the preceding claims, **characterized in that** said prosthetic element (40, 50) comprises an intraosseous taproot (10) whose central body (11) also has a triangular cross-section along at least a portion or over its entire length, as viewed in a substantially horizontal plane.

8. The kit (100) for the installation of prostheses and/or biomedical implants as claimed in one or more of the preceding claims, **characterized in that** said intraosseous taproot (10) of said prosthetic element (40, 50) comprises end sections (11a, 11b) also having a triangular cross-section as viewed in a substantially horizontal plane.

9. The kit (100) for the installation of prostheses and/or biomedical implants as claimed in the preceding claim, **characterized in that** said prosthetic element (40, 50) comprises an intraosseous taproot (10) comprising, at its front end (11a), an end element (13) having a frustoconical profile as viewed in a vertical plane.

10. The kit (100) for the installation of prostheses and/or biomedical implants as claimed in the preceding claim, **characterized in that** said prosthetic element (40, 50) comprises an intraosseous taproot (10) whose end element (13) has a width (D) in a vertical longitudinal plane that is equal to the overall footprint of the taproot (10) in the same plane.

11. The kit (100) for the installation of prostheses and/or biomedical implants as claimed in one or more of the preceding claims, **characterized in that** said intraosseous taproot element (10) comprises, at the rear end (11b) of said taproot (10) designed for connection to the prosthetic element to be associated with the bone, a base element (14) having a width (D) in a vertical longitudinal plane that is equal to the overall footprint of the taproot (10) in the same plane.

12. The kit (100) for the installation of prostheses and/or biomedical implants as claimed in one or more of the preceding claims, **characterized in that** said prosthetic element (40, 50) comprises an intraosseous taproot (10) as claimed in one or more of the preceding claims, **characterized in that** it is made in one piece with a prosthetic element (50).

13. The kit (100) for the installation of prostheses and/or biomedical implants as claimed in one or more of the preceding claims, **characterized in that** said trabeculated portion (12) of said intraosseous taproot (10) extends around said central body (11) having a triangular cross section but remains within the shape defined by said end elements (13, 14) of the taproot.

14. The kit (100) for the installation of prostheses and/or biomedical implants as claimed in one or more of the preceding claims, **characterized in that** said trabeculated portion (12) has a pore diameter that ranges from 400 to 800 microns, preferably of about 600 microns, more preferably of 640 microns.

15. The kit (100) for the installation of prostheses and/or biomedical implants as claimed in one or more of the preceding claims, **characterized in that** said trabeculated portion (12) of said intraosseous taproot (10) is formed by EBM in one piece with the central body (11).

## Patentansprüche

1. Bausatz (100) zur Installation von Prothesenkomponenten (40, 50) und/oder biomedizinischen Implantaten, wobei der Bausatz (100) eine Prothesenkomponente (40, 50) umfasst, die, ihrerseits, eine einstückig ausgebildete, intraossäre Pfahlwurzel (10) zur Befestigung der Prothesenkomponente (40, 50) am Knochen eines Patienten umfasst, wobei die Prothesenkomponente (40, 50) und die Pfahlwurzel (10) aus einem biokompatiblen Metallmaterial, insbesondere Titan oder Legierungen davon, hergestellt sind, und wobei die Pfahlwurzel (10) der Prothesenkomponente (40, 50) einen festen Zentralkörper (11), der ein axiales Durchgangsloch (15) umfasst, sowie außerhalb des Zentralkörpers (11) einen trabekulierten Abschnitt (12) umfasst, der fest mit dem Zentralkörper (11) verbunden oder einstückig mit diesem ausgebildet ist, wobei die Pfahlwurzel (10) in einer im Wesentlichen horizontalen Querschnittsebene mindestens eine Sektion mit dreieckigem Querschnitt aufweist, **dadurch gekennzeichnet, dass** der Bausatz (100) ferner mindestens eine erste Führungskomponente (20) für einen Führungsdraht, um das Einbringen eines Führungsdrahtes (K) in den Knochen zu führen sowie mindestens eine zweite Führungskomponente (30) für einen Impaktor mit dreieckigem Querschnitt umfasst.

2. Bausatz (100) zur Installation von Prothesen und/oder biomedizinischen Implantaten nach dem vorhergehenden Anspruch, **dadurch gekennzeichnet, dass** jede der Führungskomponenten (20, 30) patientenspezifisch durch Verarbeitung der Bilder einer Computertomographieaufnahme der Anatomie des Patienten hergestellt ist.

3. Bausatz (100) zur Installation von Prothesen und/oder biomedizinischen Implantaten nach dem vorhergehenden Anspruch, **dadurch gekennzeichnet, dass** jede der Führungskomponenten (20, 30) so ausgelegt ist, dass sie eindeutig mit der anatomischen Stelle der Prothesenimplantation zusammenpasst, wodurch eine korrekte Positionierung der Führungselemente und somit des Führungsdrahtes und des dreieckigen Impaktors sichergestellt wird, wodurch eine korrekte Orientierung des dreieckigen Sitzes innerhalb des Knochens sichergestellt wird, was seinerseits eine korrekte Positionierung der dreieckigen Pfahlwurzel (10) sicherstellt.

4. Bausatz (100) zur Installation von Prothesen und/oder biomedizinischen Implantaten nach dem vorhergehenden Anspruch, **dadurch gekennzeichnet, dass** die Prothesenkomponente (40, 50) und die Pfahlwurzel (10) einstückig ausgebildet sind.

5. Bausatz (100) zur Installation von Prothesen und/oder biomedizinischen Implantaten nach dem vorhergehenden Anspruch, **dadurch gekennzeichnet, dass** die Prothesenkomponente (40, 50) und die Pfahlwurzel (10) einstückig mittels additiver 3D-Techniken, nämlich EBM-Technologien, hergestellt sind.

6. Bausatz (100) zur Installation von Prothesen und/oder biomedizinischen Implantaten nach einem oder mehreren der vorhergehenden Ansprüche, **dadurch gekennzeichnet, dass** das Prothesenelement (40, 50) eine intraossäre Pfahlwurzel (10) umfasst, die entlang ihrer gesamten Länge einen dreieckigen Querschnitt aufweist, betrachtet in einer im Wesentlichen horizontalen Ebene.

7. Bausatz (100) zur Installation von Prothesen und/oder biomedizinischen Implantaten nach einem oder mehreren der vorhergehenden Ansprüche, **dadurch gekennzeichnet, dass** das Prothesenelement (40, 50) eine intraossäre Pfahlwurzel (10) umfasst, deren Zentralkörper (11) ebenfalls entlang mindestens einem Abschnitt oder über ihre gesamte Länge einen dreieckigen Querschnitt aufweist, betrachtet in einer im Wesentlichen horizontalen Ebene.

8. Bausatz (100) zur Installation von Prothesen und/oder biomedizinischen Implantaten nach einem oder mehreren der vorhergehenden Ansprüche, **dadurch gekennzeichnet, dass** die intraossäre Pfahlwurzel (10) des Prothesenelements (40, 50) Endsektionen (11a, 11b) umfasst, die ebenfalls einen dreieckigen Querschnitt aufweisen, betrachtet in einer im Wesentlichen horizontalen Ebene.

9. Bausatz (100) zur Installation von Prothesen und/oder biomedizinischen Implantaten nach dem vorhergehenden Anspruch, **dadurch gekennzeichnet, dass** das Prothesenelement (40, 50) eine intraossäre Pfahlwurzel (10) umfasst, die an ihrem vorderen Ende (11a) ein Endelement (13) mit einem kegelstumpfförmigen Profil aufweist, betrachtet in einer vertikalen Ebene.

10. Bausatz (100) zur Installation von Prothesen und/oder biomedizinischen Implantaten nach dem vorhergehenden Anspruch, **dadurch gekennzeichnet, dass** das Prothesenelement (40, 50) eine intraossäre Pfahlwurzel (10) umfasst, deren Endelement (13) in einer vertikalen Längsebene eine Breite (D) aufweist, die der gesamten Abmessung der Pfahlwurzel (10) in derselben Ebene entspricht.

11. Bausatz (100) zur Installation von Prothesen und/oder biomedizinischen Implantaten nach einem oder mehreren der vorhergehenden Ansprüche, **dadurch gekennzeichnet, dass** das intraossäre Pfahlwurzelelement (10) am hinteren Ende (11b) der Pfahlwurzel (10), das zur Verbindung mit dem mit dem Knochen zu assoziierenden Prothesenelement vorgesehen ist, ein Basiselement (14) aufweist, das in einer vertikalen Längsebene eine Breite (D) aufweist, die der gesamten Abmessung der Pfahlwurzel (10) in derselben Ebene entspricht.

12. Bausatz (100) zur Installation von Prothesen und/oder biomedizinischen Implantaten nach einem oder mehreren der vorhergehenden Ansprüche, **dadurch gekennzeichnet, dass** das Prothesenelement (40, 50) eine intraossäre Pfahlwurzel (10) nach einem oder mehreren der vorhergehenden Ansprüche umfasst, **dadurch gekennzeichnet, dass** sie einstückig mit einem Prothesenelement (50) ausgebildet ist.

13. Bausatz (100) zur Installation von Prothesen und/oder biomedizinischen Implantaten nach einem oder mehreren der vorhergehenden Ansprüche, **dadurch gekennzeichnet, dass** sich der trabekulierte Abschnitt (12) der intraossären Pfahlwurzel (10) um den Zentralkörper (11) mit dreieckigem Querschnitt herum erstreckt, jedoch innerhalb der Form verbleibt, die durch die Endelemente (13, 14) der Pfahlwurzel definiert ist.

14. Bausatz (100) zur Installation von Prothesen und/oder biomedizinischen Implantaten nach einem oder mehreren der vorhergehenden Ansprüche, **dadurch gekennzeichnet, dass** der trabekulierte Abschnitt (12) einen Porendurchmesser aufweist, der von 400 bis 800 Mikrometern reicht, vorzugsweise etwa 600 Mikrometer, bevorzugter 640 Mikrometer.

15. Bausatz (100) zur Installation von Prothesen und/oder biomedizinischen Implantaten nach einem oder mehreren der vorhergehenden Ansprüche, **dadurch gekennzeichnet, dass** der trabekulierte Abschnitt (12) der intraossären Pfahlwurzel (10) durch EBM einstückig mit dem Zentralkörper (11) gebildet ist.

## Revendications

1. Kit (100) pour l'installation de composants prothétiques (40, 50) et/ou d'implants biomédicaux, ledit kit (100) comprenant un composant prothétique (40, 50) comprenant à son tour, d'un seul tenant, une racine pivotante intra-osseuse (10) destinée à la fixation dudit composant prothétique (40, 50) à l'os d'un patient, ledit composant prothétique (40, 50) et ladite racine pivotante (10) étant réalisés en un matériau métallique biocompatible, en particulier du titane ou des alliages de celui-ci, et ladite racine pivotante (10) dudit composant prothétique (40, 50) comprenant un corps central plein (11) comportant un trou traversant axial (15) et, à l'extérieur dudit corps central (11), une partie trabéculaire (12) solidement reliée audit corps central (11) ou formée d'une seule pièce avec celui-ci, et ladite racine pivotante (10) présentant, dans un plan sensiblement horizontal de section transversale, au moins une section ayant une section transversale triangulaire, **caractérisé en ce que** ledit kit (100) comprend en outre au moins un premier composant de guidage (20) pour fil-guide destiné à guider l'insertion d'un fil-guide (K) dans l'os, et au moins un second composant de guidage (30) pour un impacteur ayant une section transversale triangulaire.

2. Kit (100) pour l'installation de prothèses et/ou d'implants biomédicaux selon la revendication précédente, **caractérisé en ce que** chacun desdits composants de guidage (20, 30) est fabriqué sur mesure pour le patient, par traitement des images issues du scanner de tomodensitométrie de l'anatomie du patient.

3. Kit (100) pour l'installation de prothèses et/ou d'implants biomédicaux selon la revendication précédente, **caractérisé en ce que** chacun desdits composants de guidage (20, 30) est conçu pour s'adapter uniquement au site anatomique d'implantation de la prothèse, garantissant ainsi le positionnement correct des éléments de guidage, et par conséquent du fil-guide et de l'impacteur triangulaire, garantissant ainsi l'orientation correcte du siège triangulaire à l'intérieur de l'os, ce qui assure à son tour le positionnement correct de la racine pivotante triangulaire (10).

4. Kit (100) pour l'installation de prothèses et/ou d'implants biomédicaux selon la revendication précédente, **caractérisé en ce que** ledit composant prothétique (40, 50) et ladite racine pivotante (10) sont réalisés d'un seul tenant.

5. Kit (100) pour l'installation de prothèses et/ou d'implants biomédicaux selon la revendication précédente, **caractérisé en ce que** ledit composant prothétique (40, 50) et ladite racine pivotante (10) sont réalisés d'un seul tenant au moyen de techniques d'impression 3D, à savoir les technologies de fusion par faisceau d'électrons.

6. Kit (100) pour l'installation de prothèses et/ou d'implants biomédicaux selon l'une ou plusieurs des revendications précédentes, **caractérisé en ce que** ledit élément prothétique (40, 50) comprend une racine pivotante intra-osseuse (10) qui présente une section transversale triangulaire le long de toute sa longueur, vue dans un plan sensiblement horizontal.

7. Kit (100) pour l'installation de prothèses et/ou d'implants biomédicaux selon l'une ou plusieurs des revendications précédentes, **caractérisé en ce que** ledit élément prothétique (40, 50) comprend une racine pivotante intra-osseuse (10) dont le corps central (11) présente également une section transversale triangulaire le long d'au moins une partie ou sur toute sa longueur, vue dans un plan sensiblement horizontal.

8. Kit (100) pour l'installation de prothèses et/ou d'implants biomédicaux selon l'une ou plusieurs des revendications précédentes, **caractérisé en ce que** ladite racine pivotante intra-osseuse (10) dudit élément prothétique (40, 50) comprend des sections d'extrémité (11a, 11b) présentant également une section transversale triangulaire, vue dans un plan sensiblement horizontal.

9. Kit (100) pour l'installation de prothèses et/ou d'implants biomédicaux selon la revendication précédente, **caractérisé en ce que** ledit élément prothétique (40, 50) comprend une racine pivotante intra-osseuse (10) comprenant, à son extrémité avant (11a), un élément d'extrémité (13) présentant un profil tronconique vu dans un plan vertical.

10. Kit (100) pour l'installation de prothèses et/ou d'implants biomédicaux selon la revendication précédente, **caractérisé en ce que** ledit élément prothétique (40, 50) comprend une racine pivotante intra-osseuse (10) dont l'élément d'extrémité (13) présente, dans un plan longitudinal vertical, une largeur (D) égale à l'empreinte totale de la racine pivotante (10) dans le même plan.

11. Kit (100) pour l'installation de prothèses et/ou d'implants biomédicaux selon l'une ou plusieurs des revendications précédentes, **caractérisé en ce que** ledit élément de racine pivotante intra-osseuse (10) comprend, à l'extrémité arrière (11b) de ladite racine pivotante (10) conçue pour être raccordée à l'élément prothétique à associer à l'os, un élément de base (14) présentant, dans un plan longitudinal vertical, une largeur (D) qui est égale à l'empreinte globale de la racine pivotante (10) dans le même plan.

12. Kit (100) pour l'installation de prothèses et/ou d'implants biomédicaux selon l'une ou plusieurs des revendications précédentes, **caractérisé en ce que** ledit élément prothétique (40, 50) comprend une racine pivotante intra-osseuse (10) selon l'une ou plusieurs des revendications précédentes, **caractérisée en ce qu'**elle est réalisée d'un seul tenant avec un élément prothétique (50).

13. Kit (100) pour l'installation de prothèses et/ou d'implants biomédicaux selon l'une ou plusieurs des revendications précédentes, **caractérisé en ce que** ladite partie trabéculaire (12) de ladite racine pivotante intra-osseuse (10) s'étend autour dudit corps central (11) présentant une section transversale triangulaire, mais reste à l'intérieur de la forme définie par lesdits éléments d'extrémité (13, 14) de la racine pivotante.

14. Kit (100) pour l'installation de prothèses et/ou d'implants biomédicaux selon l'une ou plusieurs des revendications précédentes, **caractérisé en ce que** ladite partie trabéculaire (12) présente un diamètre de pores compris entre 400 et 800 microns, de préférence d'environ 600 microns, plus préférablement de 640 microns.

15. Kit (100) pour l'installation de prothèses et/ou d'implants biomédicaux selon l'une ou plusieurs des revendications précédentes, **caractérisé en ce que** ladite partie trabéculaire (12) de ladite racine pivotante intra-osseuse (10) est formée par fusion par faisceau d'électrons d'un seul tenant avec le corps central (11).
